# EUROPÄISCHE PATENTSCHRIFT

(11) **EP 1 284 749 B1**
(45) Veröffentlichungstag und Bekanntmachung des Hinweises auf die Patenterteilung: **28.07.2004**
(21) Anmeldenummer: 01931683.5
(22) Anmeldetag: 25.05.2001
(51) Int. Cl.: A61K 38/47, A61P 9/00

(54) **VERWENDUNG VON HYALURONIDASE ZUR PROPHYLAXE UND BEHANDLUNG VON HERZ-KREISLAUF-ERKRANKUNGEN**
USE OF HYALURONIDASE FOR PREVENTING AND TREATING CARDIO VASCULAR DISEASES
UTILISATION D'HYALURONIDASE POUR LA PROPHYLAXIE ET LE TRAITEMENT DE MALADIES CARDIO-VASCULAIRES

(30) Priorität: 29.05.2000 DE 10026666
(43) Veröffentlichungstag der Anmeldung: 26.02.2003
(60) Teilanmeldung: 04009231.4
(73) Patentinhaber: Burgard, Gunther, 66424 Homburg (DE); Cullmann, Heinz Dieter, 66482 Zweibrücken (DE)
(72) Erfinder: Burgard, Gunther, 66424 Homburg (DE); Cullmann, Heinz Dieter, 66482 Zweibrücken (DE)
(74) Vertreter: Grünecker, Kinkeldey, Stockmair & Schwanhäusser Anwaltssozietät
(86) Internationale Anmeldenummer: PCT/EP2001/004940
(87) Internationale Veröffentlichungsnummer: WO 2001/091785

(56) Entgegenhaltungen:
- WO-A-99/02181
- DE-A- 19 501 378
- NL-A- 8 102 880
- US-A- 4 410 531
- NITZSCHNER H: "[Observations of the course of scleroderma after combined Hylase - carnitine therapy]. Verlaufsbeobachtungen der Sklerodermie nach kombinierter Hylase - Carnitin -Therapie." ZEITSCHRIFT FUR ARZTLICHE FORTBILDUNG, (1975 NOV 1) 69 (21) 1108. , XP001041671

## Beschreibung

Die vorliegende Erfindung betrifft die Verwendung von Hyaluronidase zur Herstellung eines Medikaments zur Prophylaxe und Behandlung von Herz-Kreislauf-Erkrankungen, wie koronarer Herzkrankheit und anderer Arteriosklerosen als auch zur Prophylaxe von Restenose bei Patienten, bei denen bereits eine Koronarintervention stattgefunden hat. Zu Koronarinterventionen zählen z.B. Stentimplantation, Ballondilatation, Rotablation und Bypassoperationen (AVKB).

Ferner betrifft die vorliegende Erfindung die Herstellung eines Medikaments zur Prophylaxe/Behandlung des akuten Herzinfarktes.

Herz-Kreislauf-Erkrankungen und die sich daraus ergebenden Störungen des Wohlbefindens und das Auftreten von körperlichen Beeinträchtigungen als Folgeerscheinung davon haben in den letzten Jahren eine zunehmende Anzahl von Menschen betroffen. Es sind mittlerweile zahlreiche Medikamente entwickelt worden, um Erkrankungen des Herzens zu behandeln. Zu diesen Kardiaka zählen komplexe Zusammensetzungen wie Pflanzenextrakte, aber auch chemische Einzelverbindungen, wie sie beispielsweise der "Roten Liste 2000" zu entnehmen sind. Wirklich befriedigende Ergebnisse bei der Behandlung von Herz-Kreislauf-Erkrankungen zeigt keines der gegenwärtig auf dem Markt befindlichen Präparate.

Die Anmeldung NL8102880 beschreibt die Verwendung von 20.000 - 80.000 IE Hyaluronidase zur Behandlung von Arteriosklerose, Durchblutungssteigerung und Embolie. Die Hyaluronidase wird hierbei über 15 - 30 min in einem Zeitintervall von 3 Wochen intraarteriell appliziert.

Der vorliegenden Erfindung lag das technische Problem zugrunde, eine weitere Möglichkeit zur Prophylaxe und Behandlung von Herz-Kreislauf-Erkrankungen des menschlichen oder tierischen Körpers anzugeben.

Dieses Problem wird erfindungsgemäß gemäß Patentanspruch 1 dadurch gelöst, daß Hyaluronidase in einer Dosierung von mindestens 6 000 Einheiten pro Tag zur Herstellung eines Medikaments zur Prophylaxe und/oder Behandlung von Herz-Kreislauf-Erkrankungen verwendet wird, wobei die Hyaluronidase i. v., vorzugsweise im Bolus z.B. innerhalb von 10 Sekunden, über einen Zeitraum von mindestens 4 Wochen zu verabreichen ist.

Weitere vorteilhafte und bevorzugte Ausführungsformen der Erfindung sind Gegenstand der Unteransprüche.

Nach einer Ausführungsform der Erfindung wird die Hyaluronidase in einer Dosierung von mindestens 12 000 Einheiten pro Tag verwendet.

Nach einer weiteren Ausführungsform der Erfindung wird die Hyaluronidase in einer Dosierung von mindestens 15 000 Einheiten pro Tag verwendet. Es kann jedoch auch mit einer Dosierung von mehr als 100.000 Einheiten pro Tag gearbeitet werden.

Nach einer weiteren Ausführungsform der Erfindung wird die Hyaluronidase außerdem in Kombination mit Carnitin und/oder CSE-Inhibitoren und/oder Nootropika und/oder durchblutungsfördernden Mitteln und/oder Vitaminen und/oder Kationen verwendet.

Es ist klar, daß die kombinierte Verwendung durch gleichzeitige Verabreichung, beispielsweise in Form eines Kombinationspräparats, oder durch unmittelbar aufeinanderfolgende oder zeitlich versetzte Verabreichung der Einzelkomponenten erfolgen kann. Selbstverständlich können das Kombinationspräparat und auch die Einzelkomponenten in Verbindung mit pharmazeutisch akzeptablen Trägern oder Verdünnungsmitteln verwendet werden.

Nach einer weiteren Ausführungsform der Erfindung erfolgt die Dosierung über mindestens 2 Monate, besonders bevorzugt mindestens 3 Monate. Dies ist jedoch nur als Beispiel zu verstehen und die konkrete Dosierungsdauer kann im Einzelfall natürlich auch varieren. Optimale Ergebnisse werden bei einer Verabreichung einer Arbeitsdosis von 12.000 Einheiten pro Tag, fünf- bis siebenmal die Woche, über 6 bis 8 Wochen erreicht.

Beispielsweise kann es sich bei der Herz-Kreislauf-Erkrankung oder -Störung um koronare Herzkrankheit (KHK), alle Formen von Arteriosklerose, Carotisstenose, Stenose der hirnzuführenden Arterien, Herzinfarkt, Apoplexie, Herzhypertrophie oder Herzverfettung handeln.
Weiterhin wird die Verwendung des Enzyms zur Herstellung eines Medikaments zur Prophylaxe von Restenose bei Patienten, bei denen bereits eine Koronarintervention stattgefunden hat, angegeben. Zu Koronarinterventionen zählen z.B. Stentimplantation, Ballondilatation, Rotablation und Bypassoperationen (AVKB).
Ferner wurde gefunden, dass erfindungsgemäß die Herstellung eines Medikaments zur Prophylaxe/Behandlung des akuten Herzinfarktes durchgeführt werden kann.

Im folgenden wird die Erfindung ohne Beschränkung detaillierter beschrieben.

Hyaluronidase ist an sich im Stand der Technik gut bekannt und gehört zu den sog. β(1-4)-Glycosidasen. Diese Enzyme werden auch als Hyaluronat-Glycanhydrolasen, EC 3.2.1.35 bis 3.2.1.36, bezeichnet. Hyaluronidase hydrolysiert Hyaluronsäure, ein lineares Heteroglykan mit alternierenden Glucuronsäure- und N-Acetylglucosamin-Resten (saures Glycosaminoglykan (Mucopolysaccharid)), und Hyaluronat (die ionische Form von Hyaluronsäure), aber auch Chondroitinsulfat.

Die Hyaluronidase kann aus beliebigen Quellen stammen und beispielsweise aus Rinderprotein (boviner Typ), Blutegeln oder Bakterien (z.B. in Form von Hyaluronat-Lyase) gewonnen werden. Auch im Stand der Technik bekannte gentechnische Verfahren können zur Herstellung von Hyaluronidase angewendet werden.

Ganz besonders bevorzugt ist eine Hyaluronidase, die Hyaluronsäure, Chondroitin-4-sulfat, Chondroitin-6-sulfat und Mukotinsulfat spaltet und somit depolymerisiert, wobei die am meisten bevorzugte Hyaluronidase ein im Handel erhältliches Enzym ist, beispielsweise die von der Firma Pharma Dessau unter der Bezeichnung Hylase® "Dessau" vertriebene Hyaluronidase.

Die Hyaluronidase kann natürlich nicht nur zur Therapie einer bereits aufgetretenen Störung bzw. Erkrankung des Herz-Kreislauf-Systems verwendet werden, sondern auch zur vorbeugenden Behandlung, d.h. der Prophylaxe der oben genannten Indikationen, um das Auftreten der Störungen zu vermeiden oder zu verzögern.

Zur Behandlung und/oder Prophylaxe der oben angegebenen Störungen kann auch ein Gemisch aus Hyaluronidasen verschiedener Herkunft eingesetzt werden.

Besonders gut geeignet ist Hyaluronidase zur Prophylaxe bei herzinfarktgefährdeten Patienten und/oder zur Behandlung der koronaren Herzkrankheit. Dabei kann das Enzym neben der i.v.-Verabreichung auch direkt intrakoronar verabreicht werden.

Die koronare Herzkrankheit ist auch unter den Begriffen "stenosierende Koronarsklerose", "degenerative Koronarerkrankung" und "ischämische Herzerkrankung" bekannt. Eine pathophysiologische Grundlage ist die durch Einengung oder Verschluß von Herzkranzgefäßen hervorgerufene Verminderung der Blutzufuhr und damit der Zufuhr von energielieferndem Substrat und Sauerstoff zum Herzmuskel, die zu einem Mißverhältnis zwischen Angebot und Bedarf führt und meist unter Belastung deutlich wird (herabgesetzte Koronarreserve). Als Krankheitsbilder sind bekannt: Angina Pectoris, Herzinfarkt sowie Linksherzinsuffizienz. Eine KHK kann auch lange symptomlos verlaufen, während im Belastungs-EKG unter Umständen Zeichen eines Durchblutungsmangels nachweisbar sind.

Der Behandlungserfolg kann z.B. durch EKG, Belastungs-EKG, Langzeit-EKG, Echokardiographie, Streßecho sowie mit Myocardszintigraphie und Koronarangiographie als auch Koronarangioskopie kontrolliert werden. Allgemein zeigte sich, daß durch die erfindungsgemäße Verabreichung die Durchblutung der Koronargefäße, teilweise bis zur vollständigen Heilung, verbessert wurde.

Die folgenden Beispiele erläutern die Erfindung, ohne sie zu beschränken.

Es wurden zahlreiche Patienten mit Herz-Kreislauf-Beschwerden behandelt, von denen einige insbesondere an koronarer Herzkrankheit litten. Die Patienten wurden mindestens 4 Wochen, vorzugsweise mindestens 2 Monate, besonders bevorzugt mindestens 3 Monate, mit einem hyaluronidasehaltigen Präparat, vorzugsweise dem Präparat Hylase® "Dessau" behandelt. Bei Verabreichung von 6 000 Einheiten pro Tag dieses Hyaluronidasepräparats, intravenös, beispielsweise fünfmal (bis siebenmal) pro Woche, wurde nach einigen Wochen eine deutliche Abnahme der Beschwerden bei den Patienten beobachtet.

Einige Patienten wurden auch mit Dosen an Hylase® "Dessau" im Bereich von 12 000 Einheiten pro Tag, fünf- bis siebenmal pro Woche, bis hin zu einer Dosis von 15 000 Einheiten pro Tag, fünf- bis siebenmal pro Woche, therapiert. Neben der vorzugsweise angewendeten i.V.-Applikation führten auch andere Arten der Applikation, wie die intramuskuläre oder subkutane, zum Erfolg, wobei die i.V.-Applikation jedoch die wirksamste Verabreichungsform war.

Bei der Verwendung anderer Hyaluronidasen als Hylase® "Dessau" können andere Dosierungen erforderlich sein, die der Fachmann aber ohne weiteres den praktischen Gegebenheiten entsprechend bestimmt.

Nach einem weiteren Aspekt der Erfindung erfolgt die Behandlung von Herz-Kreislauf-Erkrankungen mit Hyaluronidase beispielsweise in Kombination mit Carnitin. Durch Anwendung dieser Kombination kann der Herzhypertrophie und der Herzverfettung entgegengewirkt und damit das Infarktrisiko signifikant gemindert werden.

Biochemisch steht diese erfindungsgemäße Verwendung direkt in Zusammenhang mit dem Fettsäureabbau. Da die Fettsäuresynthese vorzugsweise im Cytoplasma, der oxidative Fettsäureabbau jedoch in den Mitochondrien stattfindet, müssen die Fettsäuren für den Abbau zunachst aus dem Cytoplasma in die Mitochondrien gelangen. An diesem Transport ist Carnitin (4-Trimethylamino-3-hydroxybuttersäure) beteiligt, das sowohl mit Acetyl-CoA als auch mit Acyl-CoA unter Bildung von Acetylcarnitin bzw. Acylcarnitin reagieren kann. Die Bildung der carnitinaktivierten Essigsäure bzw. Fettsäure wird durch die Acyl-CoA-Carnitintransferase katalysiert, von der zwei verschiedene Typen (für den Acetyl- bzw. Acyl-Transfer) existieren. Durch Austausch des Acetylrestes gegen einen Fettsäurerest entsteht eine Carnityl-Fettsäureverbindung, welche die Mitochondrienmembran passieren kann. Auf diese Weise erklärt sich die fördende Wirkung des Carnitins auf die Fettsäureoxidation. Nach Eintritt in das Mitochondrium wird die Fettsäure von dem Acylcarnitin auf CoA übertragen und das Carnitin steht erneut für den Fettsäuretransport zu Verfügung.

Weitere Wirkstoffe, die weder Coenzym-Funktion besitzen, noch als essentielle Bestandteile für den Menschen nachgewiesen wurden, aber unentbehrliche Zellbausteine sind und zum Teil noch unerforschte Funktionen wahrnehmen, werden als vitaminähnliche Wirkstoffe zusammengefaßt. Sie beeinflussen zum Teil die Permeabilität von Membranen und Kapillaren.

Zu ihnen gehören außer Carnitin:
- Mesoinosit (Myoinosit)
- essentielle Fettsäuren (Vitamin F)
- Flavanoide (Vitamin P)

Ein weiterer Aspekt der Erfindung betrifft die Verwendung von Hyaluronidase in Kombination mit sogenannten CSE-Hemmern (CSE = cholesterol synthesizing enzymes) wie Pravasin und allen anderen sogenannten "Statinen. Hier ist eindeutig (z.B. in der WOS-Studie) belegt, daß vor allem Pravasin und auch andere Statine eine kardioprotektive Wirkung haben. Außerdem wirken sie genau wie die Hyaluronidase der Arteriosklerose entgegen. Ziel der Kombination ist es, durch einen Synergismus der beiden Substanzen eine Optimierung der Therapie zu erreichen.

Weitere Substanzen, die die Wirkung der Hyaluronidase begünstigen können und in Kombination erfindungsgemäß angewendet werden können, sind:
1) Magnesium und andere Kationen
2) Noottropika ( z.B. Piracetam)
3) Durchblutungsfördernde Mittel (z.B. Pentoxifillin, Gingko u.a.)
4) Diverse Vitamine (z.B. Vitamin C)
5) Substanzen zur Steigerung der NO-Synthese
6) Glykoprotein-Antagonisten; wie z.B. Eptifibatid
5) Plasminogenaktivatoren (z.B. Altelase, Retelase u. a. bei akuter Koronarthrombose (Herzinfarkt).

Die erfindungsgemäße Verwendung eignet sich ferner für folgende Indikationen:
1) Cerebralsklerose
2) Multiple Sklerose
3) Onkologie: Verbesserung der Resorption von Cytostatika in erkranktem Gewebe
4) Ophthalmologie (Glaucom)
5) Diabetische Mikroangiopathie
6) Fertilitätssteigerung bei Mann und Frau

## Patentansprüche

1. Verwendung von Hyaluronidase in einer Dosierung von mindestens 6 000 Einheiten pro Tag zur Herstellung eines Medikaments zur Prophylaxe und/oder Behandlung von Herz-Kreislauf-Erkrankungen, wobei die Hyaluronidase i. v. über einen Zeitraum von mindestens 4 Wochen. zu verabreichen ist.

2. Verwendung nach Anspruch 1, **dadurch gekennzeichnet, daß** die Hyaluronidase in einer Dosierung von mindestens 12 000 Einheiten pro Tag verwendet wird.

3. Verwendung nach Anspruch 2, **dadurch gekennzeichnet, daß** die Hyaluronidase in einer Dosierung von mindestens 15 000 Einheiten pro Tag verwendet wird.

4. Verwendung nach einem der vorherigen Ansprüche, **dadurch gekennzeichnet, daß** die Hyaluronidase in Kombination mit Carnitin und/oder CSE-Inhibitoren und/oder Nootropika und/oder durchblutungsfördernden Mitteln und/oder Vitaminen und/oder Kationen verwendet wird.

5. Verwendung nach einem der vorherigen Ansprüche, **dadurch gekennzeichnet, daß** die Verabreichung über mindestens 2 Monate erfolgt.

6. Verwendung nach einem der vorherigen Ansprüche, **dadurch gekennzeichnet, daß** es sich bei der Erkrankung um koronare Herzkrankheit (KHK), Arteriosklerose, Carotisstenose, Stenose der hirnzuführenden Arterien, Herzinfarkt, Apoplexie, Herzhypertrophie oder Herzverfettung handelt.

## Claims

1. Use of hyaluronidase in a dosage of at least 6 000 units per day for the preparation of a medicament for prevention and/or treatment of cardiovascular diseases, the hyaluronidase having to be administered i. v. over a period of at least four weeks.

2. Use according to claim 1, **characterised in that** the hyaluronidase is used in a dosage of at least 12 000 units per day.

3. Use according to claim 2, **characterised in that** the hyaluronidase is used in a dosage of at least 15 000 units per day.

4. Use according to one of the previous claims, **characterised in that** the hyaluronidase is used in combination with carnitin and/or CSE inhibitors and/or nootropics and/or agents enhancing blood supply and/or vitamins and/or cations.

5. Use according to one of the previous claims, **characterised in that** the administration is carried out over at least two months.

6. Use according to one of the previous claims, **characterised in that** the disease involves coronary heart disease (CHD), arteriosclerosis, carotid stenosis, stenosis of arteries leading to the brain, cardiac infarction, apoplexy, cardiac hypertrophy, or fatty disease of the heart.

## Revendications

1. Utilisation d'hyaluronidase en une posologie d'au moins 6000 unités par jour pour la fabrication d'un médicament destiné à la prophylaxie et/ou le traitement des maladies cardio-vasculaires, dans laquelle la hyaluronidase doit être administrée par voie i.v. sur une durée d'au moins 4 semaines.

2. Utilisation selon la revendication 1, **caractérisée en ce que** la hyaluronidase est utilisée en une posologie d'au moins 12000 unités par jour.

3. Utilisation selon la revendication 2, **caractérisée en ce que** la hyaluronidase est utilisée en une posologie d'au moins 15000 unités par jour.

4. Utilisation selon l'une quelconque des revendications précédentes, **caractérisée en ce que** l'hyaluronidase est utilisée en combinaison avec la carnitine et/ou des inhibiteurs de l'ESC et/ou des nootropes et/ou des agents de fluidification du sang et/ou des vitamines et/ou des cations.

5. Utilisation selon l'une quelconque des revendications précédentes, **caractérisée en ce que** l'administration s'effectue sur au moins 2 mois.

6. Utilisation selon l'une quelconque des revendications précédentes, **caractérisée en ce que** la maladie est la cardiopathie coronarienne (CC), l'artériosclérose, la sténose carotide, la sténose des artères conduisant au cerveau, l'infarctus du myocarde, l'apoplexie, l'hypertrophie cardiaque ou le coeur gras.
